# EUROPEAN PATENT SPECIFICATION

(11) **EP 0 491 843 B1**
(45) Date of publication and mention of the grant of the patent: **11.12.1996**
(21) Application number: 90914608.6
(22) Date of filing: 13.09.1990
(51) Int. Cl.: A61K 7/32, A61K 7/34, A61K 7/38

(54) **ANTIPERSPIRANT**
SCHWEISSHEMMENDES MITTEL
PRODUIT ANTITRANSPIRATION

(30) Priority: 15.09.1989 US 408124
(43) Date of publication of application: 01.07.1992
(73) Proprietor: THE GILLETTE COMPANY, Boston, Massachusetts 02199 (US)
(72) Inventor: COE, Craig, Michael, Sagamore, MA 02532 (US); KARASSIK, Nancy, Marie, Concord, MA 01742 (US)
(74) Representative: Baillie, Iain Cameron
(86) International application number: US9005187
(87) International publication number: WO9104009

(56) References cited:
- EP-A- 0 120 210
- EP-A- 0 388 110
- EP-A- 0 388 111
- EP-A- 0 396 137
- EP-A- 0 400 546
- EP-A- 0 404 532
- FR-A- 2 242 969
- US-A- 4 511 554
- US-A- 4 832 945
- US-A- 4 840 789
- US-A- 4 853 214

## Description

This invention relates to antiperspirant products.

Antiperspirant products are well-known in the cosmetic art. One class of such products contains a suspension of an active antiperspirant ingredient, e.g., aluminum chlorohydrate, in particulate form. The antiperspirant active ingredient acts to prevent perspiration, it is believed, by going into solution in the presence of body moisture, and interacting with the sweat glands. Solid-type antiperspirants of this type typically include a gelling agent (gellant) that gives the product a solid character. These are applied by rubbing an area of the body with product to apply a layer to the skin. It is desirable that the solid product glide smoothly without flaking or crumbling. Application of antiperspirant products frequently result in objectionable aesthetic characteristics such as tackiness, and whitening, i.e., the presence of visible residues of white appearance on the skin.

Many of the desirable properties of antiperspirants can be achieved by including various ingredients to the product formulation in addition to the active agent, the vehicle and, in solids, the gelling agent. For example, surfactants may be added to ease washing of the product from the skin after use; fragrance and preservatives are commonly added; and supplemental emollients may be added to improve the application characteristics, glide, flaking, crumbling (in solids), etc. However, such additives tend to reduce the efficacy of the product. It is believed, for example, that emollients tend to encapsulate or enrobe particulates of the active ingredient and retard the dissolution and, therefore interfere with the interaction of the active antiperspirant ingredient with the sweat glands.

In accordance with the present invention, there is provided a nonaqueous solid antiperspirant composition comprising thirty to fifty percent by weight of a nonaqueous carrier vehicle; ten to thirty percent by weight of an antiperspirant active ingredient suspended in particulate form in said vehicle; a gelling agent and a masking agent to eliminate discernible whitening when the product is applied to the skin, characterized in that said masking agent comprises PPG-10 Butanediol. Said masking agent essentially eliminates discernible whitening without substantially inhibiting the antiperspirant activity of the salt.

Preferred antiperspirant products include a sufficient quantity of a gelling agent so that the antiperspirant product can be used as a solid, but a reduced amount of, or no, 'gelling agent may be used if it is desirable to make a more liquid type product.

The vehicle generally is volatile and makes up between about thirty to about fifty percent of the product and in preferred embodiments, is a volatile silicone such as cylcomethicone. Other suitable vehicles include aliphatic hydrocarbons such as isododecane, dimethicone, polyphenylmethylsiloxane liquids such as Dow Corning DC-556, SD-40 alcohol, and C₁₂₋₁₅ Alcohols Benzoate (Finsolv TN).

The active ingredient, in particulate form, is suspended in the carrier vehicle. Preferred active antiperspirant active components for use in the antiperspirant products include well-known salts such as aluminium zirconium chlorohydrates, aluminium chlorohydrates and other polyhydroxy complexes of basic aluminum salts, such as aluminum sesquichlorohydrate, aluminum dichlorohydrate, aluminum chloride, aluminum zirconium trichlorohydrate, aluminum zirconium tetrachlorohydrate, aluminum zirconium pentachlorohydrate and aluminum zirconium octachlorohydrate. A particularly preferred active ingredient is Aluminum Zirconium Tetrachlorohydrex-Gly with enhanced activity (as described in U.S. Patent No. 4,775,528). The active antiperspirant component(s) should be present in an amount effective to reduce perspiration when applied to the skin. The precise limits on the amount of active antiperspirant component that can be used will vary with the particular component and formula. As a general rule, however, the antiperspirant product should contain anywhere from ten to thirty percent (more preferably about twenty-five percent) of active antiperspirant component. The active ingredient is provided in particulate form, for ease of processing and to avoid settling from the suspension. In preferred embodiments, the products of the invention do not require conventional suspending agents such as clays and silicas (e.g., bentonite). Preferably at least fifty percent of the particulates of the antiperspirant active are of about ten µm (microns) or less in size, and more preferably at least ninety percent of the particulates of the antiperspirant active are of about ten µm (microns) or less in size (Superfine). Larger particulates may also be used.

The masking agent, in quantity less than fifty percent by weight, preferably about twenty to forty percent by weight, of the antiperspirant active salt, and five to nine pecent by weight of the total product provides essentially complete absence of discernible whitening, without substantial inhibition of antiperspirant activity. The masking agent preferably is a relatively nonvolatile emollient material (i.e., it enhances glide and smoothness of application) and, it is believed, reduces whitening by interacting with the particulates to produce an optical effect that tends to reduce light scattering and apparent whiteness, The agent does not, however, substantially impede dissolution of the antiperspirant ingredient by body fluids or the interaction of the dissolved antiperspirant ingredient with the sweat glands. PPG-10 Butanediol is available under the name MACOL 57 (Mazer Chemicals, Gurnee, Illinois).

In addition, other compatible emollients, surfactants, preservatives and fragrances may be included. Gelling agents are included for the solid compositions. Examples of suitable gelling agents include hydrogenated castor oil, fatty alcohols such as stearyl alcohol, Stearamide MEA, polyethylene-vinyl acetate copolymers, polyethylene homopolymers and blends and combinations of these. The amount of gelling agent in the solid antiperspirant product should be sufficient to cause the liquid vehicle to gel (i.e., become free-standing). In general, the solid antiperspirant product should include between about fifteen percent and about twenty-five percent (more preferably about twenty percent) of gelling agent.

Supplemental emollients generally range from one to ten percent of the product and nay be dioctyl cyclohexane, propoxylated ethers such as PPG-14 Butyl Ether, isopropyl palmitate, isopropyl myristate, myristal myristate, dimethicone, C₁₂-₁₅ Alcohol Benzoates, diisopropyl sebacate, and diisopropyl adipate. These can add to desirable aesthetic properties such as smoothness, glide, and the perception of drier and nontacky application.

In a particular embodiment, the product is a solid antiperspirant including a volatile cyclomethicone (DC-345) carrier, Superfine Aluminum-Zirconium Tetrachlorohydrex-Gly active ingredient of enhanced activity (as in U.S. Patent 4,775,528), a PPG-10 Butanediol (MACOL 57) masking agent, myristyl myristate emollient, Steareth-21 surfactant, and hydrogenated castor oil and stearyl alcohol gelling agents. The ingredients are formulated in concentrations for balanced emulsifying, stability, aesthetics and efficacy. In particular embodiments, the gelling agents may range from fifteen to twenty-five percent and supplemental emollients may range from one to ten percent. Active ingredient particles of less than ten µm (microns) size are in a solid suspension within a matrix formed substantially of the gelling agent. Dioctyl cyclohexane may be used to stabilize the suspension and prevent phase separation of the masking agent. The final product is a free standing solid suspension of active antiperspirant ingredient particles.

The following Examples illustrate representative antiperspirant products and are given by way of illustration only. The amounts in the Examples and the clams are in weight percent. The formulations of the Examples provide antiperspirant products that are characterized by essentially complete absence of discernable (to an experienced visual evaluator) whitening and provide antiperspirant efficacy of at least eighty percent of commercially available solid antiperspirant products (as measured clinically by gravimetric means). The solid products also exhibit smooth glide upon application without flaking or crumbling. The ingredients may be processed as described or conventionally known.

### Example 1 (not according to the invention)

| **CTFA NAME** | **% Active** |
|---|---|
| CYCLOMETHICONE (DC-345) | 37.7 |
| ALUMINUM ZIRCONIUM TETRACHLOROHYDREX-GLY | 23.5 |
| STEARYL ALCOHOL | 19.0 |
| HYDROGENATED POLYISOBUTENES (Panalene) | 6.0 |
| PPG-14 BUTYL ETHER (Probutyl 14) | 5.8 |
| HYDROGENATED CASTOR OIL (MP 70) | 3.5 |
| DIOCTYL CYCLOHEXANE (Cetiol S) | 3.0 |
| STEARETH-21 (Brij 721S) | 1.0 |
| FRAGRANCE | 0.3 |
| PROPYLPARABEN | 0.2 |

The Example 1 solid product is made by combining and mechanically mixing the vehicle, the masking agent and supplemental emollients until homogeneous. The active ingredient in powder form is then added, and the mixture is heated from room temperature to 60°C over a period of about one-quarter hour. At 60°C, gellant (i.e., hydrogenated castor oil and stearyl alcohol) is added and the mixture is continued to be heated to about 85°C (a temperature where the gellant has melted) with agitation to insure complete homogeneity. The mixture is cooled to about 64°C and fragrance is added. The mixture is cooled again to about 53°C and poured into suitable containers.

### Example 2 (not according to the invention)

| **CTFA NAME** | **% ACTIVE** |
|---|---|
| CYCLOMETHICONE (DC-345) | 39.0 |
| ALUMINUM ZIRCONIUM TETRACHLOROHYDREX-GLY | 23.1 |
| STEARAMIDE MEA (Monamid S) | 19.0 |
| PPG-14 BUTYL ETHER (Probutyl 14) | 8.0 |
| HYDROGENATED POLYISOBUTENES (Panalene) | 5.5 |
| TALC | 4.0 |
| STEARETH 21 (Brij 721S) | 1.0 |
| FRAGRANCE | 0.4 |

The ingredients of Example 2 are processed using a procedure similar to Example 1 with the mixture being heated to about 95°C after addition of the gellant, the temperature being held for about 15 minutes, then cooled to 80-85°C, and after fragrance is added, the mixture is stirred and immediately poured into suitable containers.

### Example 3

| **CTFA NAME** | **% ACTIVE** |
|---|---|
| CYCLOMETHICONE (DC-345) | 48.3 |
| ALUMINUM ZIRCONIUM TETRACHLOROHYDREX-GLY | 23.6 |
| STEARYL ALCOHOL | 15.2 |
| PPG-10 BUTANEDIOL (MACOL 57) | 5.0 |
| HYDROGENATED CASTOR OIL MP 70 | 2.9 |
| TALC | 2.2 |
| STEARETH-21 (Brij 721S) | 1.0 |
| PEG 8 DISTEARATE | 1.0 |
| FRAGRANCE | 0.6 |
| PROPYLPARABEN | 0.2 |
| | 100.0 |

The Example 3 solid product is made by combining and mechanically mixing the vehicle, the masking agent and supplemental emollients until homogeneous. The active ingredient in powder form is then added, and the mixture is heated from room temperature to 60°C over a period of about one-quarter hour. At 60°C, gellant is added and the mixture is continued to be heated to about 85°C (a temperature where the gellant has melted) with agitation to insure complete homogeneity. The mixture is cooled to about 64°C and fragrance is added. The mixture is cooled again to about 53°C and poured into suitable containers.

### Example 4

| **CTFA NAME** | **% ACTIVE** |
|---|---|
| CYCLOMETHICONE (DC-345) | 47.7 |
| ALUMINUM ZIRCONIUM TETRACHLOROHYDREX-GLY | 23.3 |
| STEARYL ALCOHOL | 15.0 |
| PPG-10 BUTANEDIOL (MACOL 57) | 5.0 |
| HYDROGENATED POLYISOBUTENES (Panalene) | 2.0 |
| HYDROGENATED CASTOR OIL MP 70 | 3.0 |
| TALC | 1.2 |
| STEARETH-21 (Brij 721S) | 1.0 |
| PEG 8 DISTEARATE | 1.0 |
| FRAGRANCE | 0.6 |
| PROPYLPARABEN | 0.2 |
| | 100.0 |

The Example 4 solid product is made by combining and mechanically mixing the vehicle, the masking agent and supplemental emollients until homogeneous. The active ingredient in powder form is then added, and the mixture is heated from room temperature to 60°C over a period of about one-quarter hour. At 60°C, gellant is added and the mixture is continued to be heated to about 85°C (a temperature where the gellant has melted) with agitation to insure complete homogeneity. The mixture is cooled to about 64°C and fragrance is added. The mixture is cooled again to about 53°C and poured into suitable containers.

### Example 5

| **CTFA NAME** | **% ACTIVE** |
|---|---|
| CYCLOMETHICONE (DC-345) | 45.9 |
| ALUMINUM ZIRCONIUM TETRACHLOROHYDREX-GLY | 22.7 |
| STEARYL ALCOHOL | 15.5 |
| PPG-10 BUTANEDIOL (MACOL 57) | 4.8 |
| HYDROGENATED POLYISOBUTENES (PANALENE) | 3.8 |
| HYDROGENATED CASTOR OIL MP 70 | 2.8 |
| MYRISTYL MYRISTATE | 2.0 |
| STEARETH-21 (Brig 721S) | 1.0 |
| PEG 8 DISTEARATE | 0.9 |
| FRAGRANCE | 0.6 |
| | 100.0 |

The Example 5 solid product is made by combining and mechanically mixing the vehicle, the masking agent and supplemental emollients until homogeneous. The active ingredient in powder form is then added, and the mixture is heated from room temperature to 60°C over a period of about one-quarter hour. At 60°C, gellant is added and the mixture is continued to be heated to about 85°C (a temperature where the gellant has melted) with agitation to insure complete homogeneity. The mixture is cooled to about 64°C and fragrance is added. The mixture is cooled again to about 53°C and poured into suitable containers. The product of this Example 5 has antiperspirant efficacy that is unsurpassed by commercially available solid antiperspirant products as measured clinically in a hot room by gravimetric sweat reduction determinations.

## Claims

1. A non-aqueous solid antiperspirant composition comprising thirty to fifty percent by weight of an non-aqueous carrier vehicle, ten to thirty percent by weight of an antiperspirant active ingredient suspended in particulate form in said vehicle, a gelling agent and a masking agent to eliminate discernible whitening when the composition is applied to the skin, characterized in that said masking agent is PPG-10 Butanediol.

2. The composition according to claim 1, wherein said masking agent comprises from twenty to forty percent by weight of said antiperspirant active ingredient.

3. The composition according to claim 1 or 2, wherein said carrier vehicle is cyclomethicone, an aliphatic hydrocarbon, dimethicone, a polyphonylmethylsiloxane liquid or C₁₂₋₁₅ Alcohols Benzoate.

4. The composition according to claim 3, wherein said carrier vehicle is isododecane.

5. The composition according to any of claims 1 to 4, wherein said antiperspirant active ingredient comprises aluminum zirconium chlorohydrate, aluminum chlorohydrate, or a polyhydroxy complex of a basic aluminum salt.

6. The composition according to claim 1, wherein the active ingredient is aluminum sesquichlorohydrate, aluminum dichlorohydrate, aluminum chloride, aluminum zirconium tetrachlorohydrate, aluminum zirconium pentachlorohydrate or aluminum zirconium octachlorohydrate.

7. The composition according to any of claims 1 to 6, wherein at least ninety percent of said antiperspirant active ingredient comprises particles of ten µm (microns) or less in size.

8. The composition according to any of claims 1 to 7, wherein said gelling agent comprises hydrogenated castor oil, a fatty alcohol such as stearyl alcohol, Stearamide MEA, a polyethylene-vinyl acetate copolymer, a polyethylene homopolymer or blend or combinations thereof.

9. The composition according to any of claims 1 to 8, wherein said gelling agent comprises fifteen to twenty-five percent by weight of said composition.

10. The composition according to any of claims 1 to 9, additionally comprising a supplemental emollient material.

## Patentansprüche

1. Wasserfreie, feste schweißhemmende Zusammensetzung, die 30 bis 50 Gew.-% eines wasserfreien Trägers, 10 bis 30 Gew.-% eines in disperser Form in dem Träger suspendierten schweißhemmenden Wirkstoffs, ein Geliermittel und ein Maskierungsmittel enthält, das eine erkennbare Weißfärbung verhindert, wenn die Zusammensetzung auf die Haut aufgetragen wird, dadurch gekennzeichnet, daß das Maskierungsmittel PPG-10-Butandiol ist.

2. Zusammensetzung nach Anspruch 1, bei der das Maskierungsmittel 20 bis 40 Gew.-% des schweißhemmenden Wirkstoffs enthält.

3. Zusammensetzung nach Anspruch 1 oder 2, bei der der Träger Cyclomethicon, ein aliphatischer Kohlenwasserstoff, Dimethicon, ein flüssiges Polyphenylmethylsiloxan oder ein Benzoat mit C₁₂₋₁₅-Alkoholen ist.

4. Zusammensetzung nach Anspruch 3, bei der der Träger Isododecan ist.

5. Zusammensetzung nach einem der Ansprüche 1 bis 4, bei der der schweißhemmende Wirkstoff Aluminiumzirconiumchlorhydrat, Aluminiumchlorhydrat oder einen Polyhydroxy-Komplex eines basischen Aluminiumsalzes umfaßt.

6. Zusammensetzung nach Anspruch 1, bei der der Wirkstoff Aluminiumsesquichlorhydrat, Aluminiumdichlorhydrat, Aluminiumchlorid, Aluminiumzirconiumtetrachlorhydrat, Aluminiumzirconiumpentachlorhydrat oder Aluminiumzirconiumoctachlorhydrat ist.

7. Zusammensetzung nach einem der Ansprüche 1 bis 6, bei der der schweißhemmende Wirkstoff zu mindestens 90% Teilchen mit einer Größe von höchstens 10 µm (Mikrometer) umfaßt.

8. Zusammensetzung nach einem der Ansprüche 1 bis 7, bei der das Geliermittel hydriertes Rizinusöl, einen Fettalkohol wie zum Beispiel Stearylalkohol, Stearamid MEA, ein Polyethylen-Vinylacetat-Copolymer, ein Polyethylen-Homopolymer oder Mischungen oder Kombinationen derselben umfaßt.

9. Zusammensetzung nach einem der Ansprüche 1 bis 8, bei der das Geliermittel 15 bis 25 Gew.-% der Zusammensetzung umfaßt.

10. Zusammensetzung nach einem der Ansprüche 1 bis 9, außerdem umfassend ein zusätzliches Erweichungsmittel.

## Revendications

1. Composition antiperspirante solide non aqueuse comprenant de 30 à 50 % en masse d'un véhicule support non aqueux, de 10 à 30 % en masse d'un principe actif antiperspirant mis en suspension sous forme particulaire dans ledit véhicule, un agent de gélification et un agent de masquage pour éliminer le blanchissement perceptible lorsque la composition est appliquée à la peau, caractérisée en ce que ledit agent de masquage est le PPG-10 butanediol.

2. Composition selon la revendication 1, dans laquelle ledit agent de masquage constitue 20 à 40 % en masse dudit principe actif antiperspirant.

3. Composition selon la revendication 1 ou 2, dans laquelle ledit véhicule support est la cyclométhicone, un hydrocarbure aliphatique, la diméthicone, un liquide de type polyphénylméthylsiloxane ou un benzoate d'alcools en C₁₂-C₁₅.

4. Composition selon la revendication 3, dans laquelle ledit véhicule support est l'isododécane.

5. Composition selon l'une quelconque des revendications 1 à 4, dans laquelle ledit principe actif antiperspirant comprend le chlorhydrate d'aluminium-zirconium, le chlorhydrate d'aluminium ou un complexe polyhydroxylé d'un sel d'aluminium basique.

6. Composition selon la revendication 1, dans laquelle le principe actif est le sesquichlorhydrate d'aluminium, le dichlorhydrate d'aluminium, le chlorure d'aluminium, le tétrachlorhydrate d'aluminium-zirconium, le pentachlorhydrate d'aluminium-zirconium ou l'octachlorhydrate d'aluminium-zirconium.

7. Composition selon l'une quelconque des revendications 1 à 6, dans laquelle au moins 90 % en masse dudit principe actif antiperspirant comprennent des particules ayant une dimension d'au plus 10 µm (micromètres).

8. Composition selon l'une quelconque des revendications 1 à 7, dans laquelle ledit agent de gélification comprend l'huile de ricin hydrogénée, un alcool gras tel qu'un alcool stéarylique, Stearamide MEA, un copolymère de polyéthylène-acétate de vinyle, un homopolymère de polyéthylène ou leurs mélanges ou associations.

9. Composition selon l'une quelconque des revendications 1 à 8, dans laquelle ledit agent de gélification constitue 15 à 25 % en masse de la dite composition.

10. Composition seton l'une quelconque des revendications 1 à 9, comprenant de plus un produit émollient supplémentaire.
